# EUROPEAN PATENT APPLICATION

(11) **EP 3 427 762 A1**
(43) Date of publication of application: **16.01.2019**
(21) Application number: 17763338.5
(22) Date of filing: 08.03.2017
(51) Int. Cl.: A61L 15/28, A61K 9/70, A61K 45/00, A61K 47/36

(54) **BIOABSORBABLE SHEET OR FILM**

(30) Priority: 09.03.2016 JP 2016045567
(71) Applicant: Yoshida, Yasuhiro, Sapporo-shi, Hokkaido 001-0012 (JP); Matsukawa, Akihiro, Okayama-shi, Okayama 700-0942 (JP); Okihara, Takumi, Okayama-shi, Okayama 700-0071 (JP)
(72) Inventor: Yoshida, Yasuhiro, Sapporo-shi, Hokkaido 001-0012 (JP); Matsukawa, Akihiro, Okayama-shi, Okayama 700-0942 (JP); Okihara, Takumi, Okayama-shi, Okayama 700-0071 (JP)
(74) Representative: Hoffmann Eitle
(86) International application number: PCT/JP2017/009320
(87) International publication number: WO 2017/154998

(57) **Abstract**

The present invention relates to a bioabsorbable sheet or film, composed of a composition containing phosphorylated pullulan. The bioabsorbable sheet or film of the present invention can be suitably used in the medical fields, for example, as medical adhesives to be patched to a biotissue such as organs and blood vessels during surgical operations.

## Description

### TECHNICAL FIELD

The present invention relates to a functional sheet or film which is bio-patchable, which is used in patching to an organ and a tissue in a live body.

### BACKGROUND ART

In surgical operations and the like, as the method of treatment of a wound in an organ, a blood vessel, or the like, presently, in addition to suture with a polymer suture, a method of using a bonding tape or film, an implant for a live body or the like has been mainly used.

For example, Patent Publication 1 discloses an antiadhesive film in which a laminate film comprising a first coat layer formed with a biodegradable polymer and a second coat layer formed with one or more members selected from metals, metal oxides, silicon, and silicon oxide is supported by a carrier sheet having an air permeability at a second coat layer side. Here, the laminate film is produced by spreading and drying a solution or dispersion of the polymer as a first coat layer, forming thereon a second coat layer according to a physical deposition method or a chemical deposition method, and then layering together on a carrier sheet. Moreover, upon use, the laminate film is removed from the carrier sheet, and patched so that the surface of the first coat layer contacts with a surface of a body tissue. By using the laminate film as described above, a second coating layer that does not easily soften or melt in a body would form a surface layer, whereby reducing a risk that the first coat layer is subject to accretion with other antiadhesive film.

In addition, Patent Publication 2 discloses as a film for wound curing for tissues in a live body, a film in which an ascorbic acid derivative and a specified agent having wound healing effects are supported by a sheet made of one or more biodegradable polymers selected from gelatin, starch (starch), sodium alginate, agar, cellulose, polyglutamic acid, chitin chitosan, and *konjak* jelly. As a method of forming the film, specifically, it is described that a composition in which an ascorbic acid derivative or an agent is blended with a biodegradable polymer is prepared, and the composition is then molded in accordance with a known molding method.

On the other hand, a technique of using as an antiadhesive material has been known by controlling the properties of a biodegradable polymer itself. For example, Patent Publication 3 has reported that a gelatin film which is cross-linked by a heat treatment under vacuum for a certain period of time retains its shape for a certain period of time while being degraded and absorbed in a live body, so that the gelatin film has excellent antiadhesive ability of biotissues.

### PRIOR ART PUBLICATIONS

### PATENT PUBLICATIONS

Patent Publication 1: Japanese Patent Laid-Open No. 2014-171577
Patent Publication 2: Japanese Patent Laid-Open No. 2012-213658
Patent Publication 3: Japanese Patent Laid-Open No. 2013-226166

### SUMMARY OF THE INVENTION

### PROBLEMS TO BE SOLVED BY THE INVENTION

However, conventional bioabsorbable sheets or films do not yet have sufficient strength or adhesion under wet conditions such as in the live body, so that further improvements have been desired.

An object of the present invention is to provide a bioabsorbable sheet or film having excellent strength and adhesion even under wet conditions, and also having excellent bioabsorbability, and further when the sheet or film contains a bioactive agent, the sheet or film having excellent releasability of the agent.

Also, another object of the present invention is to provide a bioabsorbable sheet or film having controlled dissolubility, or when the sheet or film contains a bioactive agent, the sheet or film having controlled sustained-release property of the agent.

### MEANS TO SOLVE THE PROBLEMS

As a result of intensive studies in order to solve the above-mentioned problems, the present inventors have found that a film obtained by using a composition containing phosphorylated pullulan as a main ingredient shows high strength and adhesion even under wet conditions, and also has excellent bioabsorbability, and further that when the composition contains a bioactive agent, the film has excellent releasability of the agent, and moreover that dissolubility, sustained-release property, or the like can be controlled by using a chemically modified phosphorylated pullulan. The present invention has been perfected thereby.

Specifically, the present invention relates to a bioabsorbable sheet or film, made of a composition containing phosphorylated pullulan or a chemically modified phosphorylated pullulan.

### EFFECTS OF THE INVENTION

The bioabsorbable sheet or film of the present invention exhibits some excellent effects that the sheet or film has excellent strength and adhesion even under wet conditions, and also has excellent bioabsorbability, and further that when the sheet or film contains a bioactive agent, the sheet or film has excellent releasability of the agent. In addition, some excellent effects are exhibited that the leakage or diffusion of the regeneration or reconstruction materials of tissues applied to a live body or the bioactive agent can be suppressed and fixed.

In addition, in the sheet or film of the present invention containing a bioactive agent, since a chemically modified phosphorylated pullulan is used, or other layer is provided, the dissolubility of the phosphorylated pullulan can be controlled, and the sustained-releasability of the agent can also be controlled.

### BRIEF DESCRIPTION OF THE DRAWINGS

FIG. 1 is a graph showing the relationship between the amount of the emulsifying agent and the film strength.
FIG. 2 is a graph showing a bioactive agent concentration at an implanted portion of a film of Example 9.
FIG. 3 is photographs showing a film after allowing to stand and after removal in the hemostatis experiment of a film of Example 9.
FIG. 4 is a photograph showing the results of sparingly solubilized state of Example 14.
FIG. 5 is a cross-sectional photograph showing a two-layer structure of Example 15.
FIG. 6 is a partially enlarged view photograph of FIG. 5.
FIG. 7 is a photograph showing a site of film after allowing to stand in Example 16.
FIG. 8 is a photograph showing a site which can expect bone formation in Example 16.

### MODES FOR CARRYING OUT THE INVENTION

The bioabsorbable sheet or film of the present invention is made of a composition containing phosphorylated pullulan (phosphorylated pullulan composition), which has a great feature in the use of phosphorylated pullulan as a biodegradable polymer. Here, the bioabsorbable sheet or film of the present invention is simply described as a sheet or film of the present invention.

### [Phosphorylated Pullulan Composition]

### [Phosphorylated Pullulan]

Phosphorylated pullulan not only has high affinity to a biotissue, but also a phosphate group thereof forms a chelate bond to a biotissue to show adsorbability, and thereafter also show bioabsorbability. In addition, the phosphorylated pullulan itself or its constituting units oligosaccharides and monosaccharides are high in biosafety, and are less likely to be metabolized by enzymes in the live body, so that there are only slight reactions with foreign matter materials. In the present invention, when the phosphorylated pullulan having the properties is applied to the live body, phosphorylated pullulan is infiltrated with a body fluid existing in the tissues or the surface of the organs in the live body (also referred to as tissue fluids), so that cations in the tissue fluids form a chelate bond with a phosphate group of the phosphorylated pullulan to form a crosslinked structure, whereby it is considered that it is made possible to exhibit viscosity over a long period of time in addition to the thickening action due to the pullulan, which in turn improves self-curing ability in the applicable sites. However, the present invention is not intended to be limited by these assumptions.

The phosphorylated pullulan can be produced by a known method of phosphorylating a hydroxyl group of pullulan. The method includes, for example, a method including reaction with sodium metaphosphate described in Carbohydrate Research 302 (1997), 27-34; a method including reaction with sodium phosphate described in Japanese Patent Laid-Open Nos. 2005-330269 and 2005-330270, and the like. Furthermore, as described in WO 87/07142, a method including reacting phosphorus pentoxide with pullulan to give phosphorylated pullulan is also favorably used. The phosphorylated pullulan obtained can be confirmed for its structure by IR analysis, NMR analysis, or the like. Here, the degree of phosphorylation of the phosphorylated pullulan can be adjusted by adjusting the amounts of raw materials used, the reaction conditions, and the like in accordance with known methods.

In addition, the phosphorylated pullulan may be partially or entirely formed into a salt, and, for example, sodium salt, potassium salt, calcium salt, magnesium salt, ammonium salt, salts with various amines, and the like are exemplified. The salt of the phosphorylated pullulan can be prepared in accordance with a known method.

The number-average molecular weight (Mn) of the phosphorylated pullulan is preferably 1,000 or more, more preferably 2,000 or more, even more preferably 5,000 or more, even more preferably 10,000 or more, and even more preferably 20,000 or more, from the viewpoint of adhesion with biotissues, strength of the sheet or film, and production costs, and the like. The upper limit is not particularly limited. In addition, the weight-average molecular weight (Mw) is preferably 10,000 or more, more preferably 20,000 or more, even more preferably 50,000 or more, even more preferably 100,000 or more, and even more preferably 200,000 or more, from the viewpoint of adhesion with biotissues, strength of the sheet or film, and production costs, and the like, and the upper limit is not particularly limited. However, if the polymerization of the phosphorylated pullulan progresses and polymerized into a higher degree, its dissolubility in water is worsened, so that it is made difficult to accurately measure the molecular weight such that it is actually undeterminable in some cases. In the present invention, such a polymer is also included as one of preferred examples. Therefore, in the present invention, the upper limit of the weight-average molecular weight of the phosphorylated pullulan is not particularly limited, and includes those that are beyond detection limit. Here, the number-average molecular weight (Mn) and the weight-average molecular weight (Mw) of the phosphorylated pullulan as used herein can be measured in accordance with the methods described in Examples set forth below.

It is desired that the phosphorylated pullulan is phosphorylated in a proportion of preferably from 0.5 to 15% by number, more preferably from 2 to 14% by number, even more preferably from 2 to 13% by number, and even more preferably from 2 to 10% by number of the hydroxyl groups, out of the entire hydroxyl groups contained in one molecule. Here, the proportion of the number of hydroxyl groups that are phosphorylated in the phosphorylated pullulan can be calculated by performing elemental analysis of phosphorylated pullulan to determine the content of phosphorus, assuming that all the determined phosphorus are derived from phosphorylated hydroxyl groups.

In the present invention, phosphorylated pullulan is used as a biodegradable polymer, and other biodegradable polymers may be used within the range that would not impair the effects of the present invention. Other biodegradable polymers are not particularly limited so long as they are known, which include natural biodegradable polymers, modified natural biodegradable polymers, synthetic biodegradable polymers, and the like. These biodegradable polymers can be used alone in a single kind or in a combination of two or more kinds.

The natural biodegradable polymers include polysaccharides, e.g., alginates, dextrans, chitin, chitosan, hyaluronic acid, celluloses, collagens, gelatins, fucoidins, starch, and glycosaminoglycans; proteins, e.g., albumin, casein, zein, and fibroins; and copolymers and blends thereof.

The modified natural biodegradable polymers include natural biodegradable polymers that are modified by synthesis. Specifically, a chemical derivative of the above natural biodegradable polymers (substitution and/or addition of a chemical group, e.g., an alkyl, an alkylene, hydroxylation, oxidation, and a combination thereof) can be used, which are exemplified by cellulose derivatives, e.g., alkyl celluloses, hydroxyalkyl celluloses, cellulose ethers, nitrocellulose. Among them, preferred examples include methyl cellulose, ethyl cellulose, hydroxypropyl cellulose, hydroxypropylmethyl cellulose, hydroxybutylmethyl cellulose, cellulose acetate, cellulose propionate, cellulose acetate butyrate, cellulose acetate phthalate, carboxymethyl cellulose, cellulose triacetate, and sodium cellulose sulfate.

The synthetic biodegradable polymers include polyhydroxy acids prepared from lactone monomers, e.g., glycolide, lactide, caprolactone, ε-caprolactone, valerolactone, and δ-valerolactone; and carbonates, e.g., trimethylene carbonate, tetramethylene carbonate, and the like; dioxanones, e.g., 1,4-dioxanone and p-dioxanone; 1, dioxepanones, e.g., 1,4-dioxepan-2-one and 1,5-dioxepan-2-one; and a combination thereof. The polymers formed therefrom include poly(lactic acid); poly(glycolic acid); poly(trimethylene carbonate); poly(dioxanone); poly(hydroxybutyric acid); poly(hydroxyvaleric acid); poly(lactide-co-(ε-caprolactone)); poly(glycolide-co-(ε-caprolactone)); poly(lactide-co-glycolic acid); polycarbonates; poly(pseudo amino acids); poly(amino acids); poly(hydroxyalkanoate)s; polyalkylene oxalates; polyoxaesters; polyanhydrides; polyorthoesters; and copolymers thereof, block copolymers thereof, homopolymers thereof, blends thereof, and combinations thereof.

In addition, besides those listed above, included are aliphatic polyesters; polyethylene glycols; glycerol; copoly(ether-ester); and copolymers thereof, block copolymers thereof, homopolymers thereof, blends thereof, and a combination thereof.

Of the biodegradable polymer usable in the sheet or film of the present invention, the content of the phosphorylated pullulan is preferably 50% by mass or more, more preferably 60% by mass or more, even more preferably 70% by mass or more, even more preferably 80% by mass or more, and even more preferably 90% by mass or more. The upper limit is not particularly limited, and the biodegradable polymer may be composed of phosphorylated pullulan.

In addition, the content of the phosphorylated pullulan in the sheet or film is preferably exceeding 30% by mass, more preferably 40% by mass or more, and even more preferably 50% by mass or more, from the viewpoint of improving adhesion. In addition, the upper limit is not particularly set, and the upper limit is usually about 90% by mass.

### [Plasticizer]

The sheet or film of the present invention can contain, as a component other than the phosphorylated pullulan, for example, a plasticizer, from the viewpoint of suppressing shrinkage during molding, thereby improving flexibility of the sheet or film.

The plasticizer includes, for example, glycerol, ethylene glycol, propylene glycol, diethylene glycol, triethylene glycol, dipropylene glycol, sorbitol, polyglycerols, polyethylene glycols, polyglycerol fatty acid esters, and the like. These plasticizers can be used alone or in a combination of two or more kinds, and it is preferable to use at least one member selected from glycerol and polyethylene glycols, from the viewpoint of flexibility of the sheet or film. Here, the molecular weight of the polyethylene glycols includes preferably from 100 to 1,000, and more preferably from 200 to 600. The plasticizer may be a synthesized product or a commercially available product.

The content of the plasticizer, based on 100 parts by mass of the phosphorylated pullulan, is preferably 1 part by mass or more, more preferably 3 parts by mass or more, and even more preferably 5 parts by mass or more, from the viewpoint of improving flexibility of the sheet or film, and the content is preferably 30 parts by mass or less, more preferably 25 parts by mass or less, and even more preferably 20 parts by mass or less, from the viewpoint of handling property of the sheet or film.

Also, the content of the plasticizer in the sheet or film is preferably 1% by mass or more, more preferably 2% by mass or more, and even more preferably 3% by mass or more, from the viewpoint of improving flexibility of the sheet or film, and the content is preferably 20% by mass or less, more preferably 15% by mass or less, and even more preferably 10% by mass or less, from the viewpoint of handling property of the sheet or film.

### [Bioactive Agent]

Since the sheet or film of the present invention is used by implanting in a live body, the sheet or film may be given with a pharmacological efficacy, and can contain a bioactive agent. When a sheet or film contains a bioactive agent, because of the interactions between an ionic group such as a phosphate group derived from the phosphorylated pullulan and a bioactive agent, the above sheet or film would also successively release the bioactive agent to be absorbed when the above sheet or film is absorbed in a live body. Accordingly, the sheet or film of the present invention can function as a base material for sustained-release property of the above bioactive agent.

The bioactive agent includes, but not particularly limited to, for example, as follows:
antibacterial agents (agents contained in the following compounds: β-lactams (penicillins, complex penicillins, β lactamase inhibitor-formulated penicillins, cephems, β lactamase inhibitor-formulated cephems, carbapenems, monobactams, penems), aminoglycosides, lincomycins, phosphomycins, tetracyclines, chloramphenicols, macrolides, ketolides, polypeptides, glycopeptides, streptogramins, quinolones, new quinolones, sulfa drugs, oxazolidinones);
anti-fungal drugs (agents contained in the following compounds: polyenes, azoles, allylamines, candins);
antiviral agents (for example, interferon, anti-herpes agents, anti-influenza agents, anti-HIV agents (including various nucleic acid-based reverse transcriptase inhibitors and nucleic acid replication inhibitors), vidarabine, ganciclovir, valganciclovir, valaciclovir, cidofovir, foscarnet, acyclovir, immunomodulators);
anti-parasitic agents (agents for nematodes (mebendazole, pyrantel pamoate, thiabendazole, diethylcarbamazine, ivermectin), agents for cestodes (niclosamide, praziquantel, albendazole), agents for trematodes (praziquantel), agents for protozoas (melarsoprol, eflornithine, metronidazole, tinidazole, miltefosine), agents for amoeba (rifampin, amphotericin B);
antiprotozoal agents (for example, antimalarial agents (quinine, chloroquine, mefloquine, fansidar, primaquine), eflornithine, furazolidone, melarsoprol, metronidazole, ornidazole, paromomycin sulfate, pentamidine, pyrimethamine, tinidazole);
nonsteroid antiflammatory agents (each of the agents contained in the following compounds: salicylic acid compounds, propionic acid compounds, acetic acid compounds, oxicams, basic compounds, pirins, non-pirins, multi-ingredient cold medicines, COX-2 inhibitor, COX-3 inhibitor);
steroid anti-inflammatory agent (for example, cortisone, predonisolone, triamcinolone, dexamethasone, betamethasone);
antihistamine agents (each of the agents contained in the following systems: ethanolamine system, propylamine system, phenothiazine system, piperazine system, second generations (epinastine, loratadine, fexofenadine, cetirizine));
prostaglandins and cytotoxic agents;
mast cell stabilizers (for example, cromolyn sodium);
receptor antagonists (for example, histamine H2 receptor antagonists, leukotriene receptor antagonists, sympathetic nerve β receptor antagonists, angiotensin II receptor antagonists, serotonin 5-HT(3) receptor antagonists, NMDA receptor antagonists, orexin receptor antagonists, adenosine a2a receptor antagonists, vasopressin receptor antagonists, ADP receptor antagonists, neurokinin 1 receptor antagonists, anticholinergic agents, cytokine receptor antagonists);
antitumor agents (each of the agents contained in the following systems: DNA crosslinking agents and alkylation agents, nitrosourea, platinum complex, metabolism antagonists (folate antagonists, purine antagonists, pyrimidine antagonists), ribonucleotide reductase inhibitors, spindle toxins (*Vinca* alkanoids, taxanes), topoisomerase inhibitors (podophyllotoxin, anthracyclines, camptothecin, tyrosine kinase inhibitors, bleomycins, mitomycin, biological reaction modifiers (interferon α), enzymes, hormone formulations, androgen receptor blockers, aromatase inhibitors, monoclonal antibodies);
immunogenic agents;
immunosuppressants (for example, cyclosporin, azathioprine, mizoribine, and FK506 (tacrolimus));
cardiovascular agents (for example, coronary vasodilators and nitroglycerin);
anti-anginal agents (for example, β-adrenaline blockers; calcium channel blockers (for example, nifedipine and diltiazem and; nitrates (for example, nitroglycerin, isosorbide dinitrate, pentaerythritol tetranitrate, and erythrityl tetranitrate);
antiarrhythmic agents (for example, bretylium tosylate, esmolol, verapamil, amiodarone, encainide, digoxin, digitoxin, mexiletine, disopyramide phosphate, procainamide, quinidine sulfate, quinidine gluconate, quinidine polygalacturonate, flecainide acetate, tocainide, and lidocaine);
antihypertensives (for example, propranolol (propanolol), propafenone, oxyprenolol, nifedipine, reserpine, trimetaphan, phenoxybenzamine, pargyline hydrochloride, deserpidine, diazoxide, guanethidine monosulfate, minoxidil, rescinnamine, sodium nitroprusside, *Rauwolfia serpentina,* alseroxylon, and phentolamine);
angiogenic agents (for example, angiocrine);
anti-angiogenic agents;
anticoagulants (for example, heparin, heparin sodium, and warfarin sodium);
antidepressants (for example, nefopam, oxypertine, doxepin, amoxapine, trazodone, amitriptyline, maprotiline, phenelzine (phenylzine), desipramine, nortriptyline, tranylcypromine, fluoxetine, doxepin, imipramine, imipramine pamoate, isocarboxazid, trimipramine, and protriptyline);
sedatives or hypnotics (for example, barbiturates (e.g., pentobarbital and secobarbital); benzodiazapines (e.g., flurazepam hydrochloride, triazolam, and midazolam));
anti-anxiety agents (for example, lorazepam, buspirone, prazepam, chlordiazepoxide, oxazepam, chlorazepate dipotassium, diazepam, hydroxyzine pamoate, hydroxyzine hydrochloride, alprazolam, droperidol, halazepam, chlormezanone, and dantrolene);
antipsychotics (for example, haloperidol, loxapine succinate, loxapine hydrochloride, thioridazine, thioridazine hydrochloride, thiothixene, fluphenazine, fluphenazine decanoate, fluphenazine enanthate, trifluoperazine, chlorpromazine, perphenazine, lithium citrate, and prochlorperazine);
anticonvulsants (for example, valproic acid, divalproex sodium, phenytoin (phenyloin), phenytoin sodium (phenyloin sodium), clonazepam, primidone, phenobarbital (phenobarbitol), carbamazepine, amobarbital sodium, methsuximide, metharbital, mephobarbital, mephenytoin (mephenyloin), phensuximide, paramethadione, ethotoin, phenacemide, secobarbital sodium (secobarbitol sodium), chlorazepate dipossasium, and trimethadione);
anti-manic agents (for example, lithium carbonate, carbamazepine, sodium valproate, clonazepam, sodium valproate);
psychoactive agents (drugs acting to mental health are considered as psychotherapeutic drugs and psychoactive agents);
tranquilizers (for example, benzodiazepine anxiolytics, etizolam, clothiazepam, alprazolam, oxazolam, prazepam, hydroxyzine hydrochloride, tandospirone citrate);
anti-migraine agents (for example, ergotamine, propranolol (propanolol), isomethepthene mucate, and dichloralphenazone, triptans);
anti-parkinson agents (for example, L-dopa and ethosuximide, profenamine hydrochloride, levodopa);
antitussives (for example, central antitussives (dihydrocodeine), adrenergic agent (trimethoxynol), xanthine derivatives (theophylline));
bronchodilators (for example, sympathomimetic agents (for example, epinephrine hydrochloride, metaproterenol sulfate, terbutaline sulfate, isoetharine, isoetharine mesylate, isoetharine hydrochloride, albuterol sulfate, albuterol, bitolterol mesylate, isoproterenol hydrochloride, terbutaline sulfate, epinephrine bitartrate, metaproterenol sulfate, epinephrine, and epinephrine bitartrate);
antiasthmatics (for example, ketotifen and traxanox);
antimuscarinic agents (for example, butylscopolamine bromide, pirenzepine hydrochloride);
anesthetics (for example, codeine, dihydrocodeinone, meperidine, morphine, and the like);
opioid receptor antagonists (for example, naltrexone and naloxone); antiemetics (for example, meclizine hydrochloride, nabilone, prochlorperazine, dimenhydrinate, promethazine hydrochloride, thiethylperazine, and scopolamine);
hypoglycemic agents (for example, human insulin, purified bovine insulin, purified porcine insulin, glyburide, chlorpropamide, glipizide, tolbutamide, and tolazamide);
antidiabetic agents (for example, biguanides and sulfonylurea derivatives);
hypolipidemic agents (for example, clofibrate, sodium dextro-thyroxine, probucol, pravastatin (pravastitin), atorvastatin (atorvastitin), lovastatin (lovastitin), and niacin);
thrombolytic agents (for example, urokinase, streptokinase, and alteplase);
anti-fibrinolytic agents (for example, aminocaproic acid);
hemorheologic agents (for example, pentoxifylline);
antiplatelet agents (for example, aspirin);
agents useful for calcium regulation (for example, calcitonin and parathyroid hormone);
agents useful for erythropoiesis stimulation (for example, erythropoietin); and
anti-arthritic agents (for example, phenylbutazone, sulindac, penicillamine (penicillanine), salsalate, piroxicam, azathioprine, indomethacin, meclofenamate, sodium aurothiomalate, ketoprofen, auranofin, aurothioglucose, and tolmetin sodium);
anti-gout agents (for example, colchicine and allopurinol);
muscle relaxants (for example, afloqualone, eperisone hydrochloride); adrenergic neuron blockers (for example, tamsulosin);
parasympathomimetic agents (for example, pilocarpine, muscarine); neurotransmitters (for example, adrenalin, gamma-aminobutyric acid, glycine);
antibodies;
gastrointestinal drugs (for example, sodium azulene sulfonate, urso, carnitine hydrochloride);
antiulcer or antireflux agents (for example, famotidine, cimetidine, ranitidine hydrochloride)
   diuretics (for example, trichlormethiazide, meticrane, furosemide);
lipids;
lipopolysaccharides;
polysaccharides;
enzymes;
decongestants (for example, pseudoephedrine, phenylephrine);
sulfonamide;
vitamins;
xanthines (for example, aminophylline, dyphylline, metaproterenol sulfate, and aminophylline);
alkaloids (for example, morphine, quinine, ephedrine);
diagnostic agents.

In addition, examples of those other than those listed above include, for example, viruses and cells; peptides, polypeptides, and proteins, and analogs, muteins, and active fragments thereof; immunoglobulins; cytokines (e.g., lymphokines, monokines, chemokines); hematopoietins; interleukins (IL-2, IL-3, IL-4, IL-6); erythropoietins; nucleases; tumor necrosis factors; colony-stimulating factors (e.g., GCSF. MCSF); insulin; tumor suppressors; blood proteins (e.g., fibrins, thrombins, fibrinogens, synthetic thrombins, synthetic fibrins, synthetic fibrinogens); gonadotropic hormone (e.g., FSH, LH, CG, and the like); hormones and hormone analogs (e.g., growth hormones); vaccines (e.g., tumoral antigens, bacterial antigens and viral antigens); somatostatin; antigens; growth factors (e.g., nerve growth factors, insulin-like growth factors,); bone morphogenetic proteins; TGF-β; protein inhibitors; protein antagonists; protein agonists; nucleic acids (e.g., antisense molecules, DNA, RNA, RNAi); oligonucleotides; polynucleotides; and ribozymes.

Furthermore, other bioactive agents include mitotane, halonitrosoureas, anthracyclines (anthrocyclines), ellipticine, ceftazidime, oxaprozin, valacyclovir, famciclovir, flutamide, enalapril, metformin (mefformin), itraconazole, gabapentin, fosinopril, tramadol, acarbose, lorazepam (lorazepan), follitropin, omeprazole, lisinopril, tramadol (tramsdol), levofloxacin, zafirlukast, granulocyte-colony stimulating factor, nizatidine, bupropion, perindopril, erbumine, adenosine, alendronate, alprostadil, betaxolol, bleomycin sulfate, dexfenfluramine, fentanyl, gemcitabine, glatiramer acetate, granisetron, lamivudine, mangafodipir trisodium, mesalamine, metoprolol fumarate, miglitol, moexipril (moexiprill), montelukast, octreotide acetate, olopatadine, paricalcitol, somatropin, sumatriptan succinate, tacrine, nabumetone, trovafloxacin, dolasetron, finasteride, isradipine, tolcapone, enoxaparin, fluconazole, lansoprazole, pamidronate, didanosine, diclofenac, cisapride, venlafaxine, troglitazone, fluvastatin, losartan, imiglucerase, donepezil, olanzapine, valsartan, fexofenadine, adapalene, doxazosin mesylate, mometasone furoate, ursodiol, felodipine, nefazodone hydrochloride, valrubicin, albendazole, medroxyprogesterone acetate, nicardipine hydrochloride, zolpidem tartrate, rubitecan, amlodipine besylate/benazepril hydrochloride, paroxetine hydrochloride, podofilox, pramipexole dihydrochloride, quetiapine fumarate, candesartan, cilexetil, ritonavir, busulfan, flumazenil, risperidone, carbamazepine, carbidopa, levodopa, ganciclovir, saquinavir, amprenavir, sertraline hydrochloride, clobustasol, diflucortolone, halobetasol propionate (halobetasolproprionate), sildenafil citrate, chlorthalidone, imiquimod, simvastatin, citalopram, irinotecan hydrochloride, sparfloxacin, efavirenz, tamsulosin hydrochloride, mofafinil, letrozole, terbinafine hydrochloride, rosiglitazone maleate, lomefloxacin hydrochloride, tirofiban hydrochloride, telmisartan, diazepam, loratadine, toremifene citrate, thalidomide, dinoprostone, mefloquine hydrochloride, trandolapril, mitoxantrone hydrochloride, tretinoin, etodolac, nelfinavir mesylate, indinavir, nifedipine, cefuroxime, and nimodipine.

These agents are contained within the range of one of ordinary skill in the art, and, for example, in embodiments, antimicrobial agents (for example, triclosan, also known as 2,4,4'-trichloro-2'-hydroxydiphenyl ether; chlorhexidine and salts thereof, including chlorhexidine acetate, chlorhexidine gluconate, chlorhexidine hydrochloride, and chlorhexidine sulfate; silver and salts thereof, including silver acetate, silver benzoate, silver carbonate, silver citrate, silver iodate, silver iodide, silver lactate, silver laurate, silver nitrate, silver oxide, silver palmitate, silver protein, and silver sulfadiazine; polymyxin; tetracycline; aminoglycosides (for example, tobramycin and gentamicin), rifampicin; bacitracin; neomycin; chloramphenicol; miconazole; quinolones (for example, oxolinic acid, norfloxacin, nalidixic acid, pefloxacin, enoxacin and ciprofloxacin); penicillins (for example, oxacillin and pipracil); nonoxynol-9; fusidic acid; and cephalosporins) may be used alone or in combination for the treatment of microbial growth. Further, the bioactive agent may include antibacterial proteins and antibacterial peptides (for example, lactoferrin and lactoferricin B)) and antibacterial polysaccharides (for example, fucans and derivatives thereof), in order to kill microbes or to prevent microbial growth.

The content of the bioactive agent cannot be unconditionally determined because the content depends upon the patients to which the sheet or film of the present invention is applied, and the content can be properly adjusted. Here, the bioactive agent may be optionally coated with a known coating agent or adsorbed to a carrier, or may be used by encapsulation with liposome, microcapsule, cyclodextrin or the like.

### [Emulsifying Agent]

In addition, the sheet or film of the present invention can contain an emulsifying agent other than the above. By including an emulsifying agent, an oil-soluble component can be contained. Also, the plasticity of the sheet or film can be controlled. In a case of combining an emulsifying agent with a bioactive agent, absorbability into a body can be controlled, such as absorption of the bioactive agent into a body is promoted with an emulsifying agent.

As the emulsifying agent, a known emulsifying agent can be used. For example, a phospholipid, an anionic surfactant, a cationic surfactant, a nonionic surfactant, or an amphoteric surfactant can be used. Specific examples include glycerol fatty acid esters, sodium lauryl sulfate, sucrose fatty acid esters, sorbitan fatty acid esters, polyoxyethylene hardened castor oil, polyoxyethylene sorbitan fatty acid esters, polyethylene glycol fatty acid esters, polyglycerol fatty acid esters, alkyl sulfonic acids, alkylbenzenesulfonic acids, polyoxyethylene alkyl ether sulfonic acids, soybean-derived lecithins, and salts thereof. These emulsifying agents can be used alone or in a combination of two or more kinds. The emulsifying agent may be a synthesized product or a commercially available product.

The content of the emulsifying agent, based on 100 parts by mass of the phosphorylated pullulan, is preferably 2 parts by mass or more, more preferably 10 parts by mass or more, and even more preferably 15 parts by mass or more, from the viewpoint of improving flexibility of the sheet or film, or from the viewpoint of dissolving or well dispersing the bioactive agent, and the content is preferably 60 parts by mass or less, more preferably 50 parts by mass or less, and even more preferably 40 parts by mass or less, from the viewpoint of handling property of the sheet or film.

In addition, the content of the emulsifying agent in the sheet or film is preferably 5% by mass or more, more preferably 10% by mass or more, and even more preferably 15% by mass or more, from the viewpoint of improving flexibility of the sheet or film, or from the viewpoint of dissolving or well dispersing the bioactive agent, and the content is preferably 35% by mass or less, more preferably 30% by mass or less, and even more preferably 25% by mass or less, from the viewpoint of handling property of the sheet or film.

Also, the sheet or film of the present invention can contain other additives besides those mentioned above. Other additives include sweeteners, flavors, colorants, pigments, preservatives, antioxidants, inorganic fillers, organic fillers, and the like. The contents of these additives can be properly adjusted within the range that would not impair the effects of the present invention, and it is desired that the content of the additives is preferably 5% by mass or less, and more preferably 1% by mass or less, when applications to live bodies are taken into consideration.

The sheet or film of the present invention may be any of those that contains a phosphorylated pullulan composition containing phosphorylated pullulan. For example, a sheet or film can be prepared by dissolving phosphorylated pullulan in a solvent, optionally adding a mixture of a plasticizer, a bioactive agent, an emulsifying agent, and various additives to spread the mixture, and drying a spread mixture to solidify. Here, a bioactive agent and an emulsifying agent may be separately mixed and then added.

The solvents that are usable may be any of those capable of dissolving phosphorylated pullulan, and the solvents include, for example, water, ethanol, acetic acid, acetone, and the like. These solvents can be used alone or in a combination of two or more kinds. Among them, water is preferred. In addition, the temperature upon dissolving is not particularly limited, and the temperature includes, for example, from 20° to 40°C. A defoaming treatment of the solution may be optionally carried out.

Also, a method for spreading a phosphorylated pullulan composition includes, for example, a method including injecting a solution of a composition into a mold to solidify, and a method including applying a solution of a composition to a support with a brush or a spatula, casting, a printing method, or the like.

Here, in the present invention, a layer of a phosphorylated pullulan composition of the present invention is laminated using a sheet as a support to produce a sheet or film of the present invention. The support sheet is removed upon use and a sheet or film made of a phosphorylated pullulan composition would be patched. The support sheet is not particularly limited, and specifically includes plastic films made of polyethylene terephthalate (PET), nylon, polyvinyl chloride, polyethylene, polypropylene, or the like, metal foils, and laminated films in which one or more films selected from these are laminated, and the like.

The drying temperature can be properly adjusted depending upon the kinds of the solvent if the solvents are to be evaporated.

In addition, the sheet or film of the present invention may be a laminate of a plurality of phosphorylated composition layers, laminated with a known other layer, or a combination thereof. When a plurality of phosphorylated pullulan composition layers are laminated, for example, a laminate can be produced by laminating layers in which the kinds of bioactive agents, the presence or absence of the agents, or contents thereof are adjusted for every layer. When a known layer is laminated, although details will be given in the section of "embodiment of providing other layer," a layer for coating containing ethyl cellulose (EC), hypromellose phthalate (HPMCP), hydroxypropylmethylcellulose acetate succinate, agar, carrageenan, Tween, or the like can be used, from the viewpoint of controlling dissolubility of the phosphorylated pullulan composition layer. The layer for coating is made of absorbable material to live bodies, and can be prepared by known techniques. For example, the layer can be prepared in the same manner as in the sheet or film of the present invention by dissolving raw materials mentioned above in water. Accordingly, one embodiment of a sheet or film of the present invention includes, for example, one in which a phosphorylated pullulan composition layer and a layer for coating are laminated in this order on the support sheet. The lamination method is not particularly limited, and, for example, each layer is separately prepared and then patched together to produce a laminate, or alternatively, each layer may be spread over already formed layer and laminated.

Thus, the sheet or film of the present invention is obtained. As the thickness of the sheet or film of the present invention, the lower limit is 10 µm, 20 µm, 30 µm, and 50 µm or so, and the upper limit is 2,000 µm, 1,500 µm, 1,200 µm, 800 µm, 500 µm, 300 µm, and 200 µm or so, and the thickness can be appropriately set depending upon the applicable sites or the releasing property of the agent when a bioactive agent is contained. Here, the thickness as used herein refers to a thickness of a phosphorylated pullulan composition layer, and when a plurality of phosphorylated pullulan composition layers are laminated, a thickness means a total thickness. In addition, in general, the term "sheet" refers to those having a thickness of from 1 to several millimeters, whereas the term "film" has thinner thickness than the sheets, a thickness of from 10 to several hundred micrometers, referring to a thin film in many cases, so that the two terms are distinctively used in the art.

The shape of the sheet or film of the present invention can be optionally determined to be circular, elliptic, multigonal-shaped (square, rectangular, rhombus, or the like), star-shaped, heart-shaped, or hatchings. Also, the area thereof cannot be unconditionally determined depending upon the thickness of the sheet or film, and the area can be adjusted depending upon the applied sites and purposes thereof. For example, the area is from 0.5 cm² to 5,000 cm² or so, and preferably from 2 cm² to 3,000 cm² or so.

The sheet or film of the present invention can be used in the restoration or regeneration of damages of organs or tissues in live bodies. Specifically, in a wounded site, a defective site, or a vulnerable site or the like in the organs in live bodies, such as the heart, the lungs, the livers, the stomach, the intestines, the gallbladder, the pancreas, the spleen, the kidneys, the urinary bladder, and the genitalia; skin; mucous membrane; nervous system tissues such as peripheral nerves and central nerves such as the brain and the spine; bones and joints, such as bones, cartilages, ligaments, and tendons; flesh, such as subcutaneous binding tissues, fascia, muscles, and adipose tissues; and vascular tissues such as blood vessels and lymphoducts, the sheet or film of the present invention can be used, when containing a support sheet, by removing a support sheet, and thereafter patching a sheet or film so that a phosphorylated pullulan composition layer directly contacts an affected site. Here, the term "in the live body or live bodies" in the present invention embraces not only a case of patching to an inner portion of live body, but also a case of patching a sheet or film to a mucous surface in the oral cavity or dermal surface or the like, or a case where one side resides in live body but the other side is exposed outside of the live body. Therefore, patching a sheet or film of the present invention to a wounded site, a defective site, a vulnerable site, or the like is intended to restore or regenerate the damages in the organs or tissues in live bodies. Accordingly, one embodiment of the present invention is to provide a coating material for a wounded site, a defective site, or a vulnerable site or the like in the organs or tissues in live bodies, that contains a sheet or film of the present invention. The coating material can be used in, for example, wound coatings, reinforcements of defective sites and vulnerable sites of the organs, reinforcements of sites that are difficult to be sutured, hemostasis (prevention of bleeding), preventing of air leakage, or the like.

In addition, when the sheet or film of the present invention contains a bioactive agent, the sheet or film can be properly used for a disease corresponding to the agent. For example, in the case of an infective disease, an infection can be calmed down by patching a film containing an agent or the like for inhibiting an infection (antibacterial agent, antiviral agent, anti-fungal agent, bactericidal agent, or the like) directly to an infected focus. In cases of epithelial tumors or non-epithelial tumors, antitumor effects can be exhibited by patching a film containing an antitumor agent (anticancer agent, antibody, low-molecular compound, or the like) to a tumor site. In a tissue damaged site or defective site, the restoration or regeneration of the tissue can be achieved by using a film containing a liquid factor such as a growth factor or a cytokine. As to inflammation sedation, pain relief and defervescence, a film containing an anti-inflammatory agent (steroid or non-steroid anti-inflammatory agent) or immunogenic agent or the like is used. As to bleeding, a film containing an anti-coagulant is patched to a bleeding site. Included are a film containing an anesthesia patched to skin or mucous membrane, which enables local anesthesia without needles, and the like. The amount of the sheet or film of the present invention used, the number of times used, and the duration of use differ depending upon the kinds of the bioactive agent contained, and the amount used is not unconditionally determined but properly set depending upon the effective amount of the bioactive agent, and purposes of use and the age, body weight, symptoms of the patients to be patched.

In another embodiment of the present invention, a sheet or film of the present invention containing the above coating material or bioactive agent is partly or entirely subjected to a surface treatment, or other layers besides the sheet or film of the present invention may be provided thereto.

### [Embodiment for Carrying out Surface Treatment]

The surface treatment includes chemical modification of a part of a hydroxyl group of phosphorylated pullulan, and the like. The chemical modification includes, for example, hydrophobic treatment of the surface, introduction of a crosslinking structure, or the like, and the above chemical modification can be carried out in accordance with a known method. The chemical modification may be any of those reactive with a hydroxyl group, which is considered to be chemically modified with an etherification agent, an esterification agent, an acetal formation agent, isocyanate, isothiocyanate, carbamoyl chloride, thiocarbamoyl chloride, a silane coupling agent, sulfonyl chloride, sulfonic acid anhydride, a polymer, other crosslinking agent for polymer, or the like. The dissolubility of phosphoryl pullulan can be controlled by these chemical modifications, thereby making it possible to control sustained-release property such as giving sustained-release property to an agent contained in a sheet or film of the present invention. Therefore, provision of a bioabsorbable sheet or film containing phosphorylated pullulan with controlled dissolubility of phosphorylated pullulan and controlled sustained-release property of an agent is one embodiment of the present invention.

The etherification agent is preferably alkyl halides such as methyl chloride and ethyl chloride; dialkyl carbonates such as dimethyl carbonate and diethyl carbonate; dialkyl sulfates such as dimethyl sulfate and diethyl sulfate; alkylene oxides such as ethylene oxide and propylene oxide; and the like. For example, ethylene oxide gas or the like is preferably used. In addition, it is not intended to limit to alkyl etherification but etherification with benzyl bromide or the like is also preferred.

The esterification agent includes carboxylic acids, acid anhydrides, carboxylic halides, ketenes, and diketenes, each of which may contain a hetero atom, and acetic acid, propionic acid, butyric acid, acrylic acid, methacrylic acid, and derivatives thereof can be used, and, for example, acetic anhydride or the like is preferred. When acetic anhydride is used, a film made of phosphorylated pullulan may be immersed in a solution of acetic anhydride, or the film may be treated with steam of acetic anhydride. In addition, as the phosphate esterification agent, phosphoryl chloride or the like can be used. The functional group to be introduced by chemical modification includes, for example, an acetyl group, a methacryloyl group, a propanoyl group, a butanoyl group an iso-butanoyl group, a pentanoyl group, a hexanoyl group, a heptanoyl group, an octanoyl group, a methyl group, an ethyl group, a propyl group, an isopropyl group, a butyl group, an iso-butyl group, a tert-butyl group, a pentyl group, a hexyl group, a heptyl group, an octyl group, and the like.

The acetal formation agent includes aldehydes. The aldehyde may be preferably those in which a hydroxyl group can be subject to an acetal reaction, without particular limitation. The aldehydes are preferably formaldehyde, acetaldehyde, propionaldehyde, normal butyraldehyde, and the like, from the viewpoint of reactivity and costs, and two or more members may be used in combination.

The isocyanate includes methyl isocyanate, ethyl isocyanate, isopropyl isocyanate, isobutyl isocyanate, trichloromethylethyl isocyanate, chloroethyl isocyanate, isopropyl isothiocyanate, isobutyl isothiocyanate, diphenylmethane isocyanate, hexamethylene diisocyanate, toluene diisocyanate, isophorone diisocyanate, and the like.

The isothiocyanate includes methyl isothiocyanate, ethyl isothiocyanate, isopropyl isothiocyanate, isobutyl isothiocyanate, chloroethyl isothiocyanate, n-octyl isothiocyanate, cyclohexyl isothiocyanate, phenyl isothiocyanate, 1,4-phenylene diisothiocyanate, and the like.

The carbamoyl chloride includes dimethyl carbamoyl chloride, diethyl carbamoyl chloride, diphenyl carbamoyl chloride, bis(2-chloroethyl) carbamoyl chloride, N-methoxy-N-methyl carbamoyl chloride, 4-morpholinyl carbamoyl chloride, and the like.

The thiocarbamoyl chloride includes dimethyl thiocarbamoyl chloride, diethyl carbamoyl chloride, diphenyl carbamoyl chloride, and the like.

As the silane coupling agent, applicable are silazanes such as vinyl silazane, hexamethyl disilazane, tetramethyl disilazane, and diphenyltetramethyl disilazane; chlorosilanes such as trimethylchlorosilane, dimethyldichlorosilane, methyltrichlorosilane, and vinyltrichlorosilane; alkoxysilanes such as trimethyl methoxysilane, dimethyl dimethoxysilane, methyl trimethoxysilane, vinyl trimethoxysilane, n-butoxytrimethylsilane, tert-butoxytrimethylsilane, sec-butoxytrimethylsilane, isobutoxytrimethylsilane, ethoxytriethylsilane, octyldimethyl ethoxysilane, or cyclohexyloxytrimethylsilane; alkoxysiloxanes such as butoxy poly(dimethyl siloxane); silane coupling agents such as vinyl triacetoxysilane, vinyl tris(methoxyethoxy)silane, vinyl trimethoxysilane, vinyl trimethoxysilane, vinyl triethoxysilane, and allyl trimethoxysilane; silyl halides such as trimethylsilyl chloride and diphenylbutyl chloride; silyltrifluoromethanesulfonates such as t-butyldimethylsilyl trifluoromethane sulfonate, and trimethylmethoxysilane, dimethyldimethoxysilane, methyltrimethoxysilane, hexamethyldisilazane, trimethylchlorosilane, and the like are preferred.

The sulfonyl chloride includes methanesulfonyl chloride, ethanesulfonyl chloride, chloromethylsulfonyl chloride, methanedisulfonyl dichloride, p-toluenesulfonyl chloride, and the like.

The sulfonic acid anhydride includes benzenesulfonic acid anhydride, trifluoromethanesulfonic acid anhydride, p-toluenesulfonic acid anhydride, and the like.

As the surface modification using a polymer, an amphophilic polymer can be used. An amphophilic polymer refers to a polymer having both a hydrophilic component and a lipophilic component in the unit of the polymer, so that the polymer has the properties of being dissolved or dispersed in both an organic solvent and water. An amphophilic polymer may be a homopolymer composed of single monomer having a hydrophilic component and a lipophilic component on its side chain, or may be a copolymerized polymer in which a hydrophilic component monomer is copolymerized with a lipophilic component monomer. The single monomer having both a hydrophilic component and a lipophilic component is, for example, (polyoxyalkylene) acrylate and methacrylate. Also, a hydrophilic component monomer may be copolymerized with a lipophilic component monomer to prepare an amphophilic polymer. In the preparation, the acrylic resins in which the method of polymerization is easy and the kinds of monomers are abundant are preferred. The acrylic resin may be a homopolymer composed only of acrylic monomer, or may be copolymerized with other monomers based on the acrylic monomer. The acrylic monomer includes general acrylic acid, methacrylic acid, methyl acrylate, methyl methacrylate, acryl chloride, methacryl chloride, or acrylic acid anhydride, and the like.

### [Embodiment for Providing Other Layers]

An embodiment for providing other layers besides the sheet or film of the present invention, i.e. a plural layer structure in which other layers are laminated, is also one embodiment of the present invention, which includes an embodiment of providing other layers on a side opposite to a patching side. By providing an embodiment as described above, the leakage of a bioactive agent in a body fluid can be suppressed when used in a live body, and evaporation of a bioactive agent can be suppressed when used for skin or the like.

Other layers include, besides the layer for coating mentioned above, layers containing biocompatible polymers or other general materials.

The biocompatible polymer includes polylactic acid (PLA), polyglycolic acid (PGA), polycaprolactone (PCL), and poly(esters) based on copolymers thereof, poly(hydroxyalkanes) such as PHB-PHV, other poly(esters), starches, cellulose, chitin, chitosan, gelatin, chondroitin sulfate and salts thereof, hyaluronic acid and salts thereof, alginic acid and salts thereof, natural polymers such as dextran, dextrin, and collagen, polycarbonate, polyurethane, polypeptide, polyethylene oxide (PEO), multi block copolymer of polyethylene oxide (PEO) and poly(butylene terephthalate) (PBT), ethylene-vinyl acetate copolymers, and the like.

Other general materials include polyethylene (PE), high-density polyethylene, medium-density polyethylene, low-density polyethylene, polypropylene (PP), polyvinyl chloride (PVC), polyvinylidene chloride, polystyrene (PS), polyvinyl acetate (PVAc), Teflon (registered trademark) (polytetrafluoroethylene, PTFE), ABS resin (acrylonitrile-butadienestyrene resin), AS resin, acrylic resin (PMMA), polyacrylonitrile, ethylene-vinyl alcohol copolymers, polyamide (PA), nylon, polyacetal (POM), modified polyphenylene ether (m-PPE, modified PPE, PPO), polybutylene terephthalate (PBT), polyethylene terephthalate (PET), glass fibers-reinforced polyethylene terephthalate (GF-PET), cyclic polyolefin (COP), polyphenylene sulfide (PPS), polytetrafluoroethylene (PTFE), polysulfonate, polyether sulfonate, amorphous polyallylate, liquid crystal polymer, polyether ether ketone, thermoplastic polyimide (PI), polyamideimide (PAI), and the like. Since these general resins are abundant in the kinds, it is possible to use not only as a single product alone but also in a combination of the kinds of resins and molecular weights in accordance with the applications.

The method for laminating other layers to a sheet or film of the present invention is not particularly limited, and the other layers can be laminated by a known method such as dry lamination, extrusion lamination, or wet lamination.

Further, the sheet or film of the present invention can be used by patching the sheet or film upon application to an organ or tissue in a live body in a manner that a regeneration or reconstruction material of a tissue in a live body or a bioactive agent is previously applied to an applicable site, and the material or the bioactive agent is coated with a phosphorylated pullulan composition layer thereon. By patching the sheet or film, some effects are exhibited that the leakage or diffusion of the regeneration or reconstruction material of the tissue in a live body or the bioactive agent is prevented, and that immobilization is facilitated. Accordingly, one embodiment of a sheet or film of the present invention includes a leakage or diffusion preventing material for regeneration or reconstruction of a tissue in a live body, or a fixing material therefor. Similarly, another embodiment includes a leakage or diffusion preventing material for a bioactive agent. Specifically, the sheet or film of the present invention can be used as artificial dura mater, antiadhesive membrane, GTR (guided tissue regeneration) membrane, scaffold, bone fixation material, adhesive material, or the like.

As the regeneration or reconstruction material of the tissue in a live body as used herein, osteoplastic materials (artificial bones (paste type, granule type)) or the like can be used. As the bioactive agent, those listed above can be used. Since the sheet or film of the present invention has excellent strength and adhesion under wet conditions, the forms of the regeneration or reconstruction material of the tissue in a live body and the bioactive agent are not particularly limited, and various forms can be used, including paste type, powders, granule type, sponge type, liquids, and the like.

### EXAMPLES

The present invention will be described more specifically by means of the following Examples. The examples are given solely for the purposes of illustration and are not to be construed as limitations of the present invention. Parts in Examples are parts by mass unless specified otherwise. Here, "ambient temperature" means 25°C.

Production Example 1 - Synthesis of Phosphorylated Pullulan Using a separable flask with an inner volume of 2 L, 40.0 g of pullulan manufactured by Hayashibara Co., Ltd. was dissolved in 200 mL of distilled water. While stirring this solution, 1,000 g of a 1 M aqueous phosphoric acid solution, a pH of which was adjusted to 5.5 with sodium hydroxide, was added thereto over 10 minutes, and after the addition the liquid mixture was continued stirring for an additional 1 hour. Thereafter, about 1,100 mL of distilled water was distilled away at a temperature between 100°C to 103°C, subsequently the residual mixture was continued stirring at 170°C for 3 hours, and a reaction product was then cooled to room temperature. The reaction product was taken out, and pulverized with a mortar, to give 98.4 g of a brown solid.

Ninety grams of the brown solid obtained above was dissolved in 1,500 mL of distilled water. While stirring this solution, 1,500 mL of a 99.5% ethanol was added thereto over 10 minutes. At the same time as the addition, the formation of the precipitates was confirmed. After the termination of addition, the mixture was continued stirring for an additional 1 hour. Thereafter, the mixture was allowed to stand to separate into layers, and the supernatant is removed by decantation method. Thereafter, the remaining precipitates are redissolved in 1,500 mL of distilled water, 1,500 mL of a 99.5% ethanol was added thereto over 10 minutes, and the precipitates were collected. The above procedures were carried out two more times, the precipitates were then dissolved in 400 mL of distilled water, and the solution was added to 2,000 mL of a 99.5% ethanol in small amounts over 5 minutes. The deposited precipitates were filtered with Kiriyama funnel (3G), washed with 500 mL of a 99.5% ethanol, and dried at 60°C under a reduced pressure for 12 hours, to give 28.5 g of a white solid with a slightly brownish color. Further, 25 g of this white solid was dissolved in distilled water, and this solution was applied to a desktop electrodialyzer MICRO ACILYZER S3, manufactured by SANACTIS, to give 13 g of phosphorylated pullulan in the form of a pale brown solid with transparency.

The solid obtained was subjected to an IR spectroscopy with FTIR-8200PC, manufactured by Shimadzu, KBr tablet method. As a result, peaks ascribed to phosphate site groups were observed at 1,000 to 1,200 cm⁻¹. In addition, ³¹P-NMR was measured with JNM-LA500 manufactured by JEOL, Ltd., and a result, signals ascribed to phosphorus of the phosphoric acid moiety, ester-bonded to pullulan were obtained at 2 to 5 ppm. An elemental analysis for phosphorus atoms was conducted according to ICP emission spectroscopy with IRIS-AP manufactured by Jarrell-Ash, and it was judged from the results that about 8.8% by number of the hydroxyl groups of the pullulan were subjected to phosphorylation. Furthermore, the GPC analysis was conducted with a column: TSKgel α-M manufactured by Tosoh Corporation, mobile phase: 0.1 M-aqueous NaCl. As a result, the solid had a number-average molecular weight (Mn) of 22,000.

### Test Example 1

The strengths of the films under wet conditions were evaluated depending upon the kinds of the biodegradable polymers.

Specifically, raw materials as listed in Table 1 were dissolved in 19 mL of distilled water, the solution obtained was defoamed with an aspirator under decompression, and the residue was spread with hands, and dried with a dryer at about 55°C, to give a film having a thickness of 70 µm. Here, as the phosphorylated pullulan a product of Production Example 1 was used, as pullulan an officinal product was used, and as collagen a product manufactured by Nitta Gelatin Inc. was used.

The physical properties of the films obtained were evaluated by a method shown below. The results are shown in Table 1.

### [Shear Stress]

A film is cut into a size of 30 × 100 mm, and both sides of the film are wetted with 10 µL of water each side. Two ends of the film are pulled with a load in a longitudinal direction, and the value is measured. Four sheets of films are used as samples, and an average is used in the evaluation.

### [Table 1]

**Table 1**

| | | Comp. Ex. 1 | Comp. Ex. 2 | Ex. 1 |
|---|---|---|---|---|
| Raw Materials, mg/strip | Pullulan | 33.6 | - | - |
| | Collagen | - | 31.5 | - |
| | Phosphorylated Pullulan | - | - | 37.5 |
| | Total | 33.6 | 31.5 | 37.5 |
| Physical Property | Shear Stress, kPa | 3.7 | 17.3 | 39.6 |

As a result, the shear adhesion is smaller under wet conditions in pullulan, so that it is clear that the film made of phosphorylated pullulan is a new bioabsorbable film showing higher adhesion under wet conditions than existing collagen films.

### Test Example 2

While an emulsifying agent is a component that can expect promotion of absorption into body, the emulsifying agent influences dissolution or dispersion of an effective ingredient, and also physical properties such as strength of a film, so that the amount thereof was studied.

Specifically, raw materials as listed in Table 2 were dissolved in 19 mL of distilled water, in the order of an emulsifying agent sucrose fatty acid ester/sorbitol = 1/4 (v/v), a plasticizer glycerol, and a phosphorylated pullulan of Production Example 1, the solution obtained was defoamed with an aspirator under decompression, and the residue was spread with hands and dried with a dryer at about 55°C, to give a film having a thickness of 70 µm.

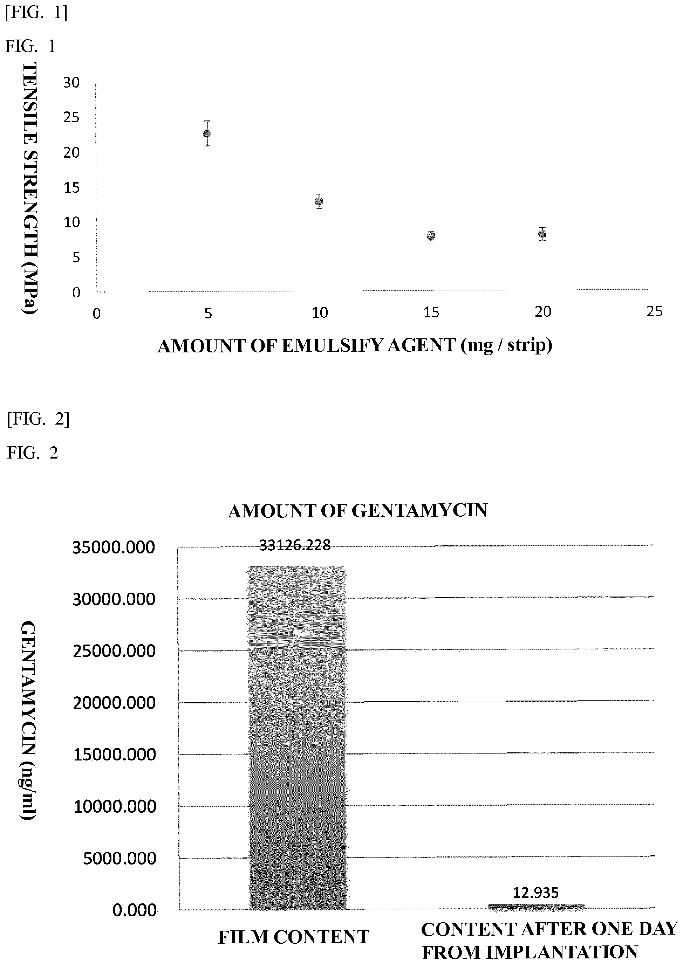

The physical properties of the films obtained were evaluated by a method shown below. The results are shown in Table 2 and FIG. 1.

### [Tensile Strength Test]

A film is cut into a size of 30 × 100 mm, and both ends of the film are sandwiched with a digital force gauge and pulled in a longitudinal direction, and the strength at a point a film breaks is defined as a tensile strength. Four sheets of films are used as samples, and an average is used in the evaluation. It is shown that the larger the tensile strength, the higher the film strength, and if a tensile strength is 2 MPa or more, it is no problem in the use for patching.

### [Dissolution Test]

A film cut into a size of 16.5 × 22 mm is allowed to float in a petri dish containing water, to observe a manner of dissolving in a static state, and a complete dissolution time is measured. Four sheets of films are used as samples, and an average is used in the evaluation. It is shown that the longer the dissolution time, the higher the dissolubility of the film, and if the dissolution time is 30 to 45 seconds or so, it is no problem in use in live bodies.

### [Table 2]

**Table 2**

| | | Ex. 2 | Ex. 3 | Ex. 4 | Ex. 5 |
|---|---|---|---|---|---|
| Raw Materials, mg/strip | Plasticizer | 2.5 | 2.5 | 2.5 | 2.5 |
| | Emulsifying Agent | 5 | 10 | 15 | 20 |
| | Phosphorylated Pullulan | 37.5 | 37.5 | 37.5 | 37.5 |
| | Total | 45 | 50 | 55 | 60 |
| Physical Properties | Tensile Strength, MPa | 22.7 | 12.8 | 7.8 | 8.0 |
| | Dissolution Time, sec | 43 | 40 | 35 | 32 |

As a result, it could be seen that the film strength tends to be reduced along with the increase in the amount of the emulsifying agent, saturating at 8 MPa or so. The aiming film strength is about 2 MPa, suggesting that sufficient strength can be maintained even when an emulsifying agent is added. Also, in the dissolution test, the dissolution was found at around 40 seconds regardless of the amount of the emulsifying agent.

### Test Example 3

In a case of a film containing a bioactive agent, whether or not a film can be formed was studied.

Specifically, raw materials as listed in Table 3 were dissolved in 19 mL of distilled water, in the order of an emulsifying agent sucrose fatty acid ester/sorbitol = 1/4 (v/v), and a plasticizer glycerol, a bioactive agent was then mixed therewith, and a phosphorylated pullulan of Production Example 1 was further added thereto to dissolve. The solution obtained was defoamed with an aspirator under decompression, and the residue was spread with hands and dried with a dryer at about 55°C, to give a film having a thickness of 70 µm. Here, as the bioactive agent, calcium chloride manufactured by Tomita Pharmaceutical Co., Ltd., estradiol manufactured by ZHEJIANG XIANJU PHARMACEUTICAL CO.,LTD., raloxifene manufactured by LKT Laboratories, Inc., arginine manufactured by AJINOMOTO CO., INC., or gentamycin manufactured by Yantai Zinchu Pharmaceutical Limited were used.

The physical properties of the films obtained were evaluated by Test Example 2 and a method shown below. The results are shown in Table 3.

### [Removal Test]

A phenolic resin is wetted with physiological saline, and a bottom half of a film cut to a size of 1.5 cm width and 10 cm in length is patched to the phenolic resin and dried. The phenolic resin is sandwiched between a digital force gauge, a side not bonded is pulled in a 180-degree-direction, and a strength at a point that is completely removed from the phenolic resin is defined as an adhesive strength. Four sheets of films are used as samples, and an average is used in the evaluation. It is shown that the larger the adhesive strength, the greater the adhesion of the film, and if an adhesive strength is 2.0 N/15 mm width or so, it is no problem in use in patching.

### [Table 3]

**Table 3**

| | | Ex. 3 | Ex. 6 | Ex. 7 | Ex. 8 | Ex. 9 | Ex.10 |
|---|---|---|---|---|---|---|---|
| Raw Materials, mg/strip | Calcium Chloride | - | 8 | - | - | - | - |
| | Estradiol | - | - | 1 | - | - | - |
| | Raloxifene | - | - | - | 1 | - | - |
| | Arginine | - | - | - | - | 8 | - |
| | Gentamycin | - | - | - | - | - | 1 |
| | Plasticizer | 2.5 | 2.5 | 2.5 | 2.5 | 2.5 | 2.5 |
| | Emulsifying Agent | 10 | 10 | 10 | 10 | 10 | 10 |
| | Phosphorylated Pullulan | 37.5 | 37.5 | 37.5 | 37.5 | 37.5 | 37.5 |
| | Total | 50 | 58 | 51 | 51 | 58 | 51 |
| Physical Properties | Tensile Strength, MPa | 12.8 | 9.8 | 8.5 | 8.4 | 11.6 | 10.5 |
| | Dissolution Time, sec | 40 | 41 | 40 | 42 | 45 | 41 |
| | Adhesive Strength, N/15 mm Width | 1.96 | 2.14 | 2.25 | 1.85 | 2.06 | 1.86 |

As a result, it was found out that by the addition of the bioactive agent, the tensile strength is lowered as compared to that of placebo (Example 3), and the degree of lowering is greater in cases of estradiol (Example 7) and raloxifene (Example 8) as compared to others. However, this is considered to be due to the matter that the bioactive agent is formulated in a dispersed state, and the film strengths in both are sufficiently higher than an intended level (2 MPa or so), so that it was clear that a film with a sufficient strength is obtained even when the bioactive agent is added. Also, in the dissolution test, the dissolution was found around 40 seconds or so regardless of the addition of the bioactive agent. It was found that the adhesive strengths nearly fulfill a target value of 2.0 N / 15 mm width or so in all the compositions.

### Test Example 4

In a case of a film containing a bioactive agent, the influences by emulsification of the bioactive agent were studied.

Specifically, with respect to the same components as in Test Example 3, the test example was conducted in the same manner as in Test Example 3 except that the bioactive agent and the emulsifying agent were previously mixed to be emulsified and then mixed, to give a film having a thickness of 70 µm.

The physical properties of the films obtained were evaluated in the same manner as in Test Example 3. The results are shown in Table 4.

### [Table 4]

**Table 4**

| | | Ex. 3 | Ex. 6 | Ex. 7 | Ex. 8 | Ex. 9 | Ex. 10 |
|---|---|---|---|---|---|---|---|
| Raw Materials, mg/strip | Calcium Chloride | - | 8 | - | - | - | - |
| | Estradiol | - | - | 1 | - | - | - |
| | Raloxifene | - | - | - | 1 | - | - |
| | Arginine | - | - | - | - | 8 | - |
| | Gentamycin | - | - | - | - | - | 1 |
| | Plasticizer | 2.5 | 2.5 | 2.5 | 2.5 | 2.5 | 2.5 |
| | Emulsifying Agent | 10 | 10 | 10 | 10 | 10 | 10 |
| | Phosphorylated Pullulan | 37.5 | 37.5 | 37.5 | 37.5 | 37.5 | 37.5 |
| | Total | 50 | 58 | 51 | 51 | 58 | 51 |
| Physical Properties | Tensile Strength, MPa | 12.8 | 8.5 | 7.4 | 7.5 | 10.5 | 8.7 |
| | Dissolution Time, sec | 40 | 37 | 36 | 42 | 40 | 39 |
| | Adhesive Strength, N/15 mm Width | 1.96 | 1.97 | 1.94 | 1.54 | 2.16 | 1.73 |

As a result, it was clarified that no significant influences were given in the physical properties of the film even when an emulsified bioactive agent was used.

### Test Example 5 - Pharmacological Test 1

A film of Example 10 in a size of 10 × 10 mm was implanted subcutaneously to a back portion on dorsolumbar muscle of a C57BL/6 mouse. After 24 hours later, the mouse was killed, and the tissues of the implanted part were extracted. The gentamycin agent concentration in the tissue was measured according to ELISA method one day after implantation. Here, as a reference, a film of Example 10 was allowed to stand in 1 mL of PBS overnight, the supernatant was collected the following day, and the gentamycin agent concentration was measured in the same manner (film content). The results are shown in FIG. 2. Here, it was confirmed during the film implantation that the film immediately adapted to the tissue, and adhesion is shown when pinched with a pair of tweezers.

As a result, the gentamycin agent concentration was 33,126 ng/mL for one dissolved in PBS, and the concentration in the tissue after one day from implantation was 13 ng/mL, so that it is considered that the agent is absorbed in the body after one day from implantation.

### Test Example 6 - Hemostatis Experiment

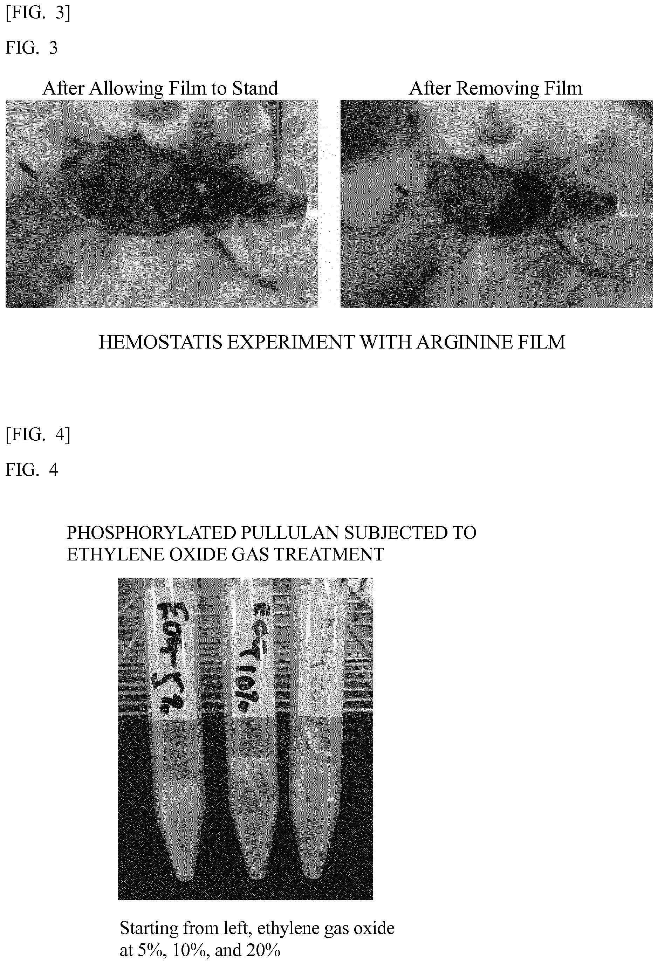

As to the arginine-containing film obtained in Example 9, the hemostasis effect was studied (FIG. 3). The liver surface of a mouse was rubbed to cause damage, and an arginine-containing film was placed thereon and allowed to stand. By allowing the film to stand, the bleeding was likely to be reduced visually. Even if the film was removed immediately thereafter, the bleeding could not be confirmed from the liver surface.

### Example 11

A 30 L reaction vessel was charged with 26 L of ultrapure water and 350 g of a food additive pullulan manufactured by Hayashibara Co., Ltd. (based on charged amounts). Thereto was added 581 mL of a 50 w/v% aqueous sodium hydroxide at an internal temperature of from 20° to 25°C, and the mixture was stirred in the same temperature range for 19 hours. The internal temperature was cooled to 0°C, and 229 g of phosphoryl chloride was added thereto at 0° to 10°C. The mixture was stirred in the same temperature range for 1 hour, the internal temperature was then adjusted to 20° to 25°C, and the mixture was stirred for 15 hours. The mixture was subjected to a membrane filtration concentration with a UF membrane, the mixture was concentrated until a distillate was reduced to a small amount, and ultrapure water was added thereto, to provide an aqueous phosphorylated pullulan solution. The aqueous phosphorylated pullulan solution obtained was powdered with a spray-dryer, to give 280 g of phosphorylated pullulan. Since it is not completely dissoluble in water, its weight-average molecular weight could not be determined.

### Example 12

A 1,000 L GL reactor was charged with 508 L of ion-exchanged water and 7 kg of a food additive pullulan manufactured by Hayashibara Co., Ltd. (based on charged amounts). The previously prepared aqueous sodium hydroxide with 5.81 kg of sodium hydroxide and 18 kg of ion-exchanged water was added thereto at an internal temperature of 20° to 25°C, the mixture was stirred in the same temperature range for 5 hours, and 4.56 kg of phosphoryl chloride was added dropwise thereto at an internal temperature of from 0° to 10°C. The mixture was stirred in the same temperature range for 1 hour, and an internal temperature was adjusted to 20° to 25°C. An aqueous sodium hydroxide was further added thereto, and the mixture was stirred overnight. The mixture was subjected to a membrane filtration concentration with a UF membrane, the mixture was concentrated until a distillate was reduced to a small amount, and ultrapure water was added thereto, to provide an aqueous phosphorylated pullulan solution. The aqueous phosphorylated pullulan solution obtained was powdered with a spray-dryer, to give 3.5 kg of phosphorylated pullulan having a weight-average molecular weight of 210,110.

The weight-average molecular weight of Example 12 is measured under the following conditions.

### < Measurement Conditions >

Measurement equipment: high-performance liquid chromatograph Column: TSK gel GMPWXL (7.8 mm ID × 390 mm) × connecting two columns
Mobile phase: 200 mM aqueous sodium nitrate solution
Flow rate: 1 mL/min
Detector: refractive index detector
Column temperature: 40°C
Amount injected: 100 µL
Sample solution: 2 mg/mL
(prepared by adding 5 mL of the mobile phase to 10 mg of the sample, and shaking the mixture to dissolve)
Measurement: A calibration curve is drawn with pullulan preparation products, and a weight-average molecular weight is calculated therefrom.

### Example 13 - Control Example 1 of Dissolubility

The amount 0.0236 g of a phosphorylated pullulan film produced in Example 6 was treated with a steam of acetic anhydride heated to 120°C for 5 minutes. An untreated phosphorylated pullulan film was swollen and dissolved when immersed in ultrapure water for 2 hours. On the other hand, the phosphorylated pullulan treated with acetic anhydride was swollen to a mass of 0.0916 g, and dried to have a mass of 0.0176 g. When this was immersed in water, an emulsifying agent or the like was undesirably eluted, so that as a phosphorylated pullulan film flexibility was slightly lost but the shape of film form was maintained. It could be seen from the above that the phosphorylated pullulan film can be made sparingly soluble by the acetic anhydride treatment. Here, it can similarly be made sparingly soluble by treatment with phosphoryl chloride in place of acetic anhydride.

### Example 14 - Control Example 2 of Dissolubility

The powder of the phosphorylated pullulan was subjected to sterilization with an ethylene oxide gas. The amount of the ethylene oxide gas reacted was changed by changing the exposure time to the gas, and the changes were observed. As a result, it could be seen that as the exposure time to the ethylene oxide gas progresses, the phosphorylated pullulan is modified, and from the matter that the phosphorylated pullulan obtained became sparingly soluble to water, it could be seen that the phosphorylated pullulan can be made sparingly soluble to water by the treatment with an ethylene oxide gas. The results are shown in FIG. 4. The numbers 5%, 10%, and 20% in the figure show concentrations of an ethylene oxide gas.

### Example 15 - Production Example of Two-Layer Structure

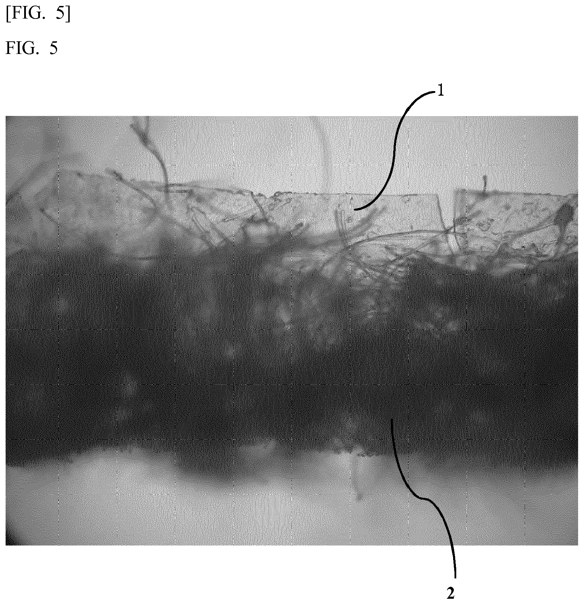

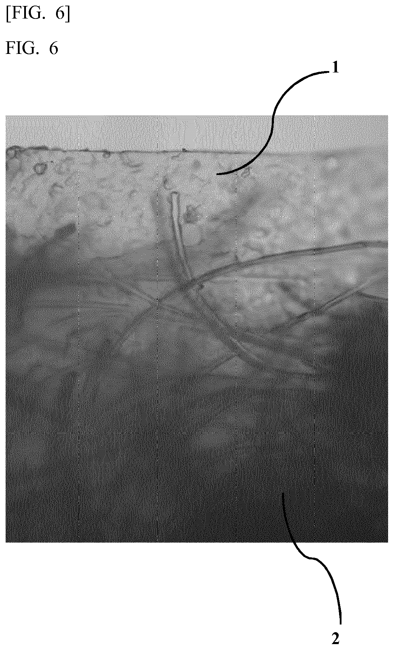

A phosphorylated pullulan film produced in the same manner as in Example 5 was pasted together with a polyglycolic acid nonwoven fabric under the product name of NEOVEIL, having a thickness of 0.3 mm, in accordance with a known wet lamination method, to produce a two-layer structure sheet of a phosphorylated pullulan layer and a polyglycolic acid nonwoven fabric. The results are shown in FIGs. 5 and 6.

### Example 16 - Bone Formation Model

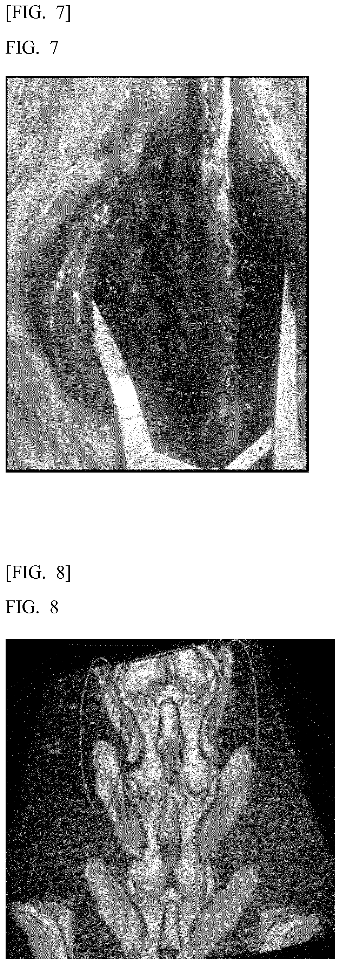

Vertebral transverse processes of rats were drilled out, and a phosphorylated pullulan film having a size of 5 mm each side containing BMP-2 which was prepared in the same manner except for formulating 1 µg/cm² of BMP-2 was allowed to stand at the same site in a position of FIG. 7. The film was immediately adsorbed to the tissues, thereby making it difficult to remove. The bone formation can be expected in encircled sites in FIG. 8.

### INDUSTRIAL APPLICABILITY

The bioabsorbable sheet or film of the present invention can be suitably used in the medical fields, for example, as medical adhesives to be patched to a biotissue such as organs and blood vessels during surgical operations.

### EXPLANATION OF NUMERALS

- 1: Phosphorylated pullulan layer
- 2: Polyglycolic acid nonwoven fabric

## Claims

1. A bioabsorbable sheet or film, comprising a composition comprising phosphorylated pullulan.

2. The sheet or film according to claim 1, wherein the composition further comprises a plasticizer.

3. The sheet or film according to claim 2, wherein the plasticizer comprises at least one member selected from glycerol and polyethylene glycol.

4. The sheet or film according to any one of claims 1 to 3, wherein the composition further comprises a bioactive agent.

5. The sheet or film according to any one of claims 1 to 4, which is supported by a supporting sheet.

6. The sheet or film according to any one of claims 1 to 5, which is a coating agent for a wound site, a defective site, or a vulnerable site in an organ or tissue in a live body.

7. The sheet or film according to any one of claims 1 to 5, which is a leakage or diffusion preventing material or a fixing material for regeneration or reconstruction of a tissue in a live body.

8. The sheet or film according to any one of claims 1 to 5, which is a leakage or diffusion preventing material of a bioactive agent.

9. A bioabsorbable sheet or film, comprising a composition comprising a chemically modified phosphorylated pullulan.

10. The sheet or film according to claim 9, wherein the chemical modification is a chemical modification using a compound selected from the group consisting of etherification agents, esterification agents, acetal formation agents, isocyanates, isothiocyanates, carbamoyl chloride, thiocarbamoyl chloride, silane coupling agents, sulfonyl chlorides, sulfonic acid anhydrides, polymers, and crosslinking agents for polymers.

11. The sheet or film according to claim 9 or 10, wherein the chemical modification is surface hydrophobic treatment of the phosphorylated pullulan.

12. The sheet or film according to claim 9 or 10, wherein the chemical modification is introduction of a crosslinked structure into phosphorylated pullulan.

13. The sheet or film according to any one of claims 9 to 12, wherein the composition further comprises a bioactive agent.

14. The sheet or film according to claim 13, wherein the sustained-release property of the bioactive agent is controlled.

15. The sheet or film according to any one of claims 1 to 14, comprising a multi-layered structure in which other layers are laminated thereto.
